# EUROPEAN PATENT APPLICATION

(11) **EP 4 174 173 A1**
(43) Date of publication of application: **03.05.2023**
(21) Application number: 20941553.8
(22) Date of filing: 25.06.2020
(51) Int. Cl.: C12N 5/0793, C12N 5/071, C12M 3/00, C12M 1/32

(54) **BRAIN ORGANOID MANUFACTURING METHOD**

(71) Applicant: Next & Bio Inc., Seoul 08826 (KR)
(72) Inventor: CHUNG, Seok, Seoul 04024 (KR); YANG, Ji Hoon, Seoul 04413 (KR); NA, Kyu Hwan, Busan 48791 (KR); JUNG, Yong Hun, Seoul 02860 (KR)
(74) Representative: Kador & Partner Part mbB
(86) International application number: PCT/KR2020/008280
(87) International publication number: WO 2021/261623

(57) **Abstract**

The present invention provides a method of producing brain organoids.

## Description

### [Technical Field]

The present invention relates to a method of producing brain organoids. More specifically, the present invention relates to a method of producing brain organoids without using a hydrogel.

### [Background Art]

A process of returning differentiated somatic cells to cells in an undifferentiated state (for example, stem cells) refers to reprogramming. Induced pluripotent stem cells (iPSCs) are also called reverse-differentiated stem cells and reverse-differentiated pluripotent stem cells, and reprogramming is the conversion of somatic cells into stem cells using reprogramming factors (Oct4, Klf4, Sox2, c-Myc, and the like) (Non-Patent Documents 1 and 2).

In the treatment of neurological diseases such as Alzheimer's disease, Parkinson's disease, cerebral infarction, cerebral hemorrhage, and spinal cord injury, a variety of new therapeutic candidate materials through nerve cell regeneration have emerged, but solutions for screening these therapeutic candidate materials are still insufficient.

Recently developed organoid technology utilizes a 3D culture method. Korean Patent No. 10-1756901 (Patent Document 1) discloses a cell culture chip capable of culturing 3D tissue cells. In the cell culture chip of Patent Document 1, a first culture part, a second culture part, and the third culture part are formed in each layer, and the degree of cell growth progress can be confirmed in each layer. However, the cell culture chip of Patent Document 1 has a problem in that spheroids and/or organoids cannot be obtained in high yield.

Further, there is a case where the pipetting work of replacing a culture solution during cell culture is performed, and in the case of a Corning spheroid microplate capable of 3D cell culture, spheroids or organoids in cell culture are affected, so that there is a problem which is not good for the cell culture environment because there is a case where the spheroids or organoids are sucked up or the positions thereof are changed during the pipetting work.

Matrigel (product name of BD Biosciences) is a protein complex extracted from sarcoma cells of Engelbreth-Holm-Swarm (EHS) mice, and contains an extracellular matrix (ECM) such as a laminin, collagen and a heparan sulfate proteoglycan, and a growth factor such as a fibroblast growth factor (FGF), an epiderma growth factor (EFG), an insulin-like growth factor (IGF), transforming growth factor-beta (TGF-β), and a platelet-derived growth factor (PDGF). The complex which forms Matrigel is utilized as a substrate for cell culture by providing a complex extracellular environment found in many tissues.

Since Matrigel is derived from mouse sarcoma, there is a high risk of transferring an immunogen and a pathogen. In addition, although Matrigel is used for cell growth and tissue formation, there is also criticism that there is a big problem with cell reproducibility because Matrigel is such a complex material. It is also unclear whether Matrigel simply acts as a passive 3D scaffold which provides a physical support for growing organoids, or whether Matrigel actively affects organoid formation by providing a biologically essential element. Furthermore, Matrigel is also expensive. Therefore, although Matrigel is a material which has contributed to the development of the cell culture technology field, there is also a fact that the development of the technology field is hindered by Matrigel.

Lancaster et al. have made brain organoids from human-induced pluripotent stem cells (Non-Patent Document 3). However, since this brain organoid culture uses Matrigel and is performed by putting the brain organoid in a large incubator, there is a disadvantage in that a large amount of medium should be used and the sizes are made differently. Moreover, in the case of the brain organoids produced in this manner, there is a problem in that various organs (cerebrum, midbrain, retina, and the like) are mixed in one organoid and do not come out uniformly, and the sizes are also different.

Thus, the present inventors have conducted continuous studies on a technology of producing brain organoids from induced pluripotent stem cells without using a hydrogel, thereby completing the present invention.

### [Related Art Documents]

### [Patent Document]

1. Korean Patent No. 10-1756901

### [Non-Patent Documents]

1. Takahashi K, Yamanaka S. Induction of pluripotent stem cells from mouse embryonic and adult fibroblast cultures by defined factors. Cell. 2006; 126:663-676.
2. Takahashi K, Tanabe K, Ohnuki M, Narita M, Ichisaka T, Tomoda K, Yamanaka S. Induction of pluripotent stem cells from adult human fibroblasts by defined factors. Cell. 2007;131:861-872.
3. Madeline A. Lancaster, Juergen A. Knoblich, Generation of Cerebral Organoids from Human Plurpotent Stem Cells, Nat Protoc. 2014 October ; 9(10): 2329-2340.

### [Disclosure]

### [Technical Problem]

An object of the present invention is to provide a method of producing brain organoids.

However, a technical problem to be achieved by the present invention is not limited to the aforementioned problems, and other problems that are not mentioned may be clearly understood by those skilled in the art from the following description.

### [Technical Solution]

To achieve the object, the present invention provides a method of producing brain organoids, the method including:
i) culturing somatic cells;
ii) preparing a hydrogel-free 3D cell culture plate for producing induced pluripotent stem cells;
iii) producing induced pluripotent stem cells by reprogramming the cultured somatic cells into the induced pluripotent stem cells in the hydrogel-free 3D cell culture plate;
iv) isolating the induced pluripotent stem cells from the 3D cell culture plate of step iii);
v) preparing a 3D cell culture plate which is not coated with a hydrogel for forming brain organoids; and
vi) forming brain organoids by culturing the isolated induced pluripotent stem cells in a hydrogel-free 3D cell culture plate,
wherein the 3D cell culture plate includes:
a well plate including a plurality of main wells and a plurality of sub wells formed at lower portions of the main wells to be injected with a cell culture solution and including recessed parts on a bottom surface thereof; and a connector for large-capacity and high-speed high content screening (HCS), which supports the well plate, and
the connector for high content screening (HCS) includes a base equipped with a fixing means so as to be attached to and detached from a lower end of the well plate and a cover positioned on an upper portion of the well plate to be coupled to the base, the main well has a step formed so as to be tapered at a predetermined site, and the step has an inclination angle (θ) ranging from 10 to 60° with respect to a wall of the main well.

The somatic cells may be fibroblasts, but are not limited thereto, and any somatic cells known in the art can be used.

The somatic cells may be cultured in a general 2D well plate, a 3D cell culture plate, or a 3D plate according to the present invention.

The forming of the brain organoids may include,
after making the induced pluripotent stem cells into an embryonic body,
inducing neuroepithelial cells by adding a neuroepithelial induction medium to the aggregated induced pluripotent stem cells;
differentiating the neuroepithelial cells into a neuroectodermal tissue by adding a neuroectodermal differentiation medium thereto;
proliferating a neuroepithelial bud by adding a neuroepithelial bud induction medium to the neuroectodermal tissue; and
forming a brain tissue by adding a brain tissue induction medium to the proliferated neuroepithelial bud.

The embryonic body means a process of transforming induced pluripotent stem cells or embryonic stem cells into a spheroid form in order to differentiate the induced pluripotent stem cells or embryonic stem cells into other cells.

The hydrogel may be an extracellular matrix-based hydrogel.

The extracellular matrix-based hydrogel may be Matrigel (product name).

The brain organoid may have a size of 0.8 to 1.3 mm. The brain organoid may have a size of preferably 1 mm or less.

The sub well of the 3D cell culture plate may have an inclined surface formed so as to taper toward the recessed part, the sub wells may have an upper end diameter ranging from 3.0 to 4.5 mm, the recessed parts may have an upper end diameter ranging from 0.45 to 1.5 mm, an inclined surface (θ₂) between the sub well and the recessed part may range from 40 to 50°, and a length ratio of the diameter of the sub wells to the diameter of the recessed parts may range from 1:0.1 to 0.5.

The main wells of the 3D cell culture plate may have an individual volume ranging from 100 to 300 µl, the recessed parts may have an individual volume ranging from 20 to 50 µl, and an individual volume ratio of the main well to the recessed part may be 1 : 0.1 to 0.5 on average.

The main well includes a space part between the step and the sub well, the space part may have a height (aₕ) ranging from 2.0 to 3.0 mm on average, the sub well may have a height (bₕ) from 1.0 to 2.0 mm on average, and a height ratio (aₕ:bₕ) of the space part to the sub well may range from 1:0.3 to 1.

Hereinafter, the present invention will be described in detail.

Since the present invention may be modified in various forms and include various exemplary embodiments, specific exemplary embodiments will be illustrated in the drawings and described in detail in the Detailed Description.

However, the description is not intended to limit the present invention to the specific embodiments, and it is to be understood that all the changes, equivalents and substitutions included in the idea and technical scope of the present invention are included in the present invention. When it is determined that the detailed description of the related publicly known art in describing the present invention may obscure the gist of the present invention, the detailed description thereof will be omitted.

The terms used in the present application are used only to describe specific embodiments, and are not intended to limit the present invention. Singular expressions include plural expressions unless the context clearly indicates otherwise.

In the present invention, the term "include" or "have" is intended to indicate the presence of the characteristic, number, step, operation, constituent element, part or any combination thereof described in the specification, and should be understood that the presence or addition possibility of one or more other characteristics or numbers, steps, operations, constituent elements, parts or any combination thereof is not pre-excluded.

In general, when cells, spheroids, organoids, and the like are cultured, a hydrogel is used to serve as an extracellular matrix. In general, when induced pluripotent stem cells are reprogrammed using a 2D plate or a 3D cell culture plate, the cell culture plate is coated with an extracellular matrix-based hydrogel (for example, Matrigel) and used.

However, the present invention provides a method of producing induced pluripotent stem cells using a hydrogel-free 3D cell culture plate, and producing brain organoids using the induced pluripotent stem cells. First, a specific description on the 3D cell culture plate of the present invention is as follows.

In an exemplary embodiment, the present invention uses a 3D cell culture plate including:
a well plate including a plurality of main wells and a plurality of sub wells formed at lower portions of the main wells to be injected with a cell culture solution and including recessed parts on a bottom surface thereof; and
a connector for large-capacity and high-speed high content screening (HCS), which supports the well plate,
wherein the connector for high content screening (HCS) includes a base equipped with a fixing means so as to be attached to and detached from a lower end of the well plate and a cover positioned on an upper portion of the well plate to be coupled to the base,
the main well has a step formed so as to be tapered at a predetermined site, and the step has an inclination angle (θ) ranging from 10 to 60° with respect to a wall of the main well.

A 96-well plate in the related art has a problem in that it takes a lot of time and costs because experiments and analyses should be performed several times or more in order to evaluate the efficacy of a drug in high yield. Furthermore, there is a case where the pipetting work of replacing a culture solution during cell culture is often performed, and in the case of a Corning spheroid microplate in the related art, spheroids or organoids in cell culture are affected, so that there was a problem which is not good for the cell culture environment because there is a case where spheroids or organoids are sucked up or the positions thereof are changed during the pipetting work.

Therefore, the present invention has been made in an effort to solve the above-described problems, and provides a cell culture plate capable of manufacturing spheroids/organoids in high yield by including a plurality of sub wells in a plurality of main wells in a well plate, and capable of uniformly capturing images in the well plate by including a connector for large-capacity high-speed high content screening (HCS), which supports the well plate to reduce a tolerance when a large-capacity and high-speed image is captured. Furthermore, the present invention provides a cell culture plate capable of minimizing the effects of the pipetting work during replacement of a medium on cells to be cultured by the step of the main well.

Hereinafter, preferred exemplary embodiments of the present invention will be described in detail with reference to the accompanying drawings. Prior to the description, terms or words used in the specification and the claims should not be interpreted as being limited to a general or dictionary meaning and should be interpreted as a meaning and a concept which conform to the technical spirit of the present invention based on a principle that an inventor can appropriately define a concept of a term in order to describe his/her own invention by the best method.

Accordingly, since the exemplary embodiments described in the present specification and the configurations illustrated in the drawings are only the most preferred exemplary embodiments of the present invention and do not represent all of the technical spirit of the present invention, it is to be understood that various equivalents and modified examples, which may replace the exemplary embodiments and the configurations, are possible at the time of filing the present application.

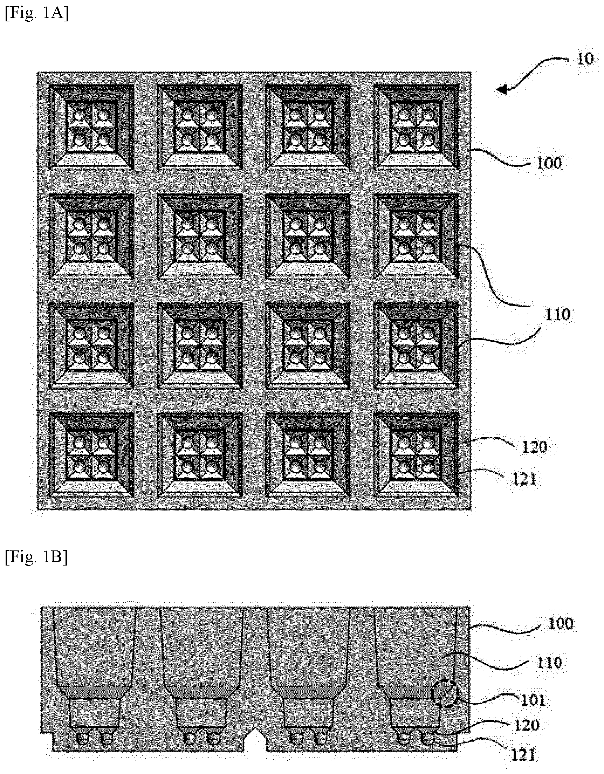

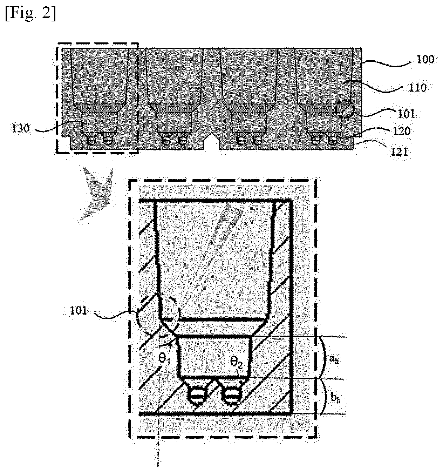

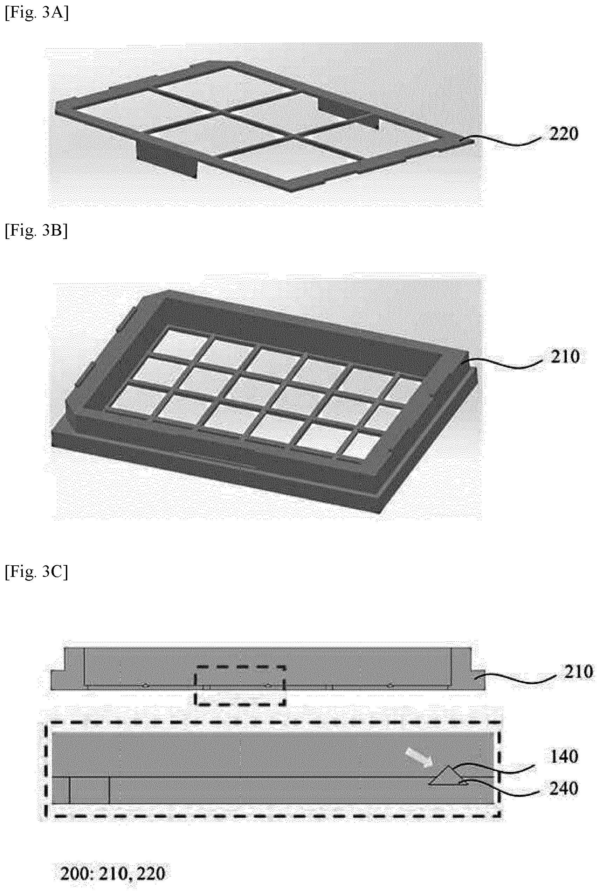

FIG 1A is a front view of a cell culture plate according to an exemplary embodiment of the present invention , FIG 1B is a cross-sectional view of the cell culture plate according to an exemplary embodiment of the present invention, FIG 2 is a view illustrating a main well formed in the cell culture plate according to an exemplary embodiment of the present invention, and FIG 3 is a view illustrating a well plate, a base and a cover of the cell culture plate according to an exemplary embodiment of the present invention ((A) a cover, (B) a base, and (C) a fixing means of a microplate and a base).

Hereinafter, a cell culture plate according to an exemplary embodiment of the present invention will be described in detail with reference to FIGS. 1 to 3.

As illustrated in FIGS. 1 to 3, a cell culture plate 10 according to an exemplary embodiment of the present invention includes a well plate 100 including a plurality of main wells 110 and a plurality of sub wells 120 formed at lower portions of the main wells 110 to be injected with a cell culture solution and including recessed parts 121 on a bottom surface thereof; and a connector 200 for large-capacity and high-speed high content screening (HCS), which supports the well plate 100.

First, the well plate 100 according to an exemplary embodiment of the present invention will be described in detail.

The well plate 100 is made into a plate shape that is plastic injection-molded through a mold. In order to manufacture a mold for plastic injection as described above, the main well 110 has a repeating pattern as a well structure such that the unit cost of production can be reduced and the size can be easily increased using fine machining. Therefore, cells are easily mass-produced, and the cells can be transformed into various sizes according to the user's requirements and used.

A plurality of the main wells 110 is formed in the well plate 100, and each main well 110 includes a step 101. The step 101 is formed at a predetermined site of the main well 110, and more specifically, the step 101 may be formed at a position which is 1/3 to 1/2 of a total length of the main well 110, and the step 101 may be formed at a position which is 1/3 to 1/2 of the total length from the lower end of the main well 110.

In the related art, there is a case where the pipetting work of replacing a culture solution during cell culture is performed, and in this case, spheroids or organoids in cell culture are affected, so that there is a problem which is not good for the cell culture because there is a case where the spheroids or organoids are sucked up or the positions thereof are changed during the pipetting work, but the step 101 is provided to prevent this problem.

The step 101 may be a space to which a pipette is applied, and specifically, may have an inclination angle (θ) ranging from 10 to 60° with respect to a wall of the main well 110. Alternatively, the step 101 may have an inclination angle ranging from 20 to 50°, preferably ranging from 30 to 45°. When the inclination angle of the step 101 is less than 10°, the inclination angle within the main well 110 is so small that the space to which a pipette can be applied is not sufficient, and as a result, when the culture solution in the main well 110 is sucked up, the pipette may slide inside the sub well 120, causing spheroids or organoids to be sucked up, or the positions thereof, and the like to be changed. Furthermore, when the inclination angle (θ) exceeds 60°, a space to which a pipette can be applied is provided, but the inclination angle of the step 101 is so large that it may be difficult to sufficiently suck up the culture solution, and when cells are seeded on the sub well 120, a problem in that cells are seeded on the step 101 without entering all the sub wells 120 may occur. Therefore, it is desirable to have an inclination angle in the above-described range.

Meanwhile, the main well according to an exemplary embodiment of the present invention may include a space part 130 between the step 101 and a sub well 120 to be described below. Specifically, the space part 130 is a space into which a culture solution is injected, and is a space in which cells inside the sub well 120 can share the same culture solution.

More specifically, the space part 130 may have a height (aₕ) ranging from 2.0 to 3.0 mm on average, or ranging from 2.2 to 2.8 mm, or ranging from 2.3 to 2.7 mm on average. Furthermore, the sub well 120 may have a height (bₕ) ranging from 1.0 to 2.0 mm on average, or ranging from 1.2 to 1.8 mm on average.

For example, the space part 130 may have a height (aₕ) of 2.5 mm on average, and the sub well may have a height (bₕ) of 1.5 mm on average.

In this case, a height ratio (aₕ:bₕ) of the space part to the sub well 120 may range from 1:0.3 to 1, and more specifically, a height ratio (aₕ:bₕ) of the space part to the sub well 120 may be 1:0.4 to 0.9 or 1:0.5 to 0.8. When a ratio of the height of the space part to the height of the sub well 120 is less than 1:0.3, the cells in culture may escape from the inside even with a small force during the exchange of the media of the sub well 120, and when a ratio of the height of the space part to the height of the sub well 120 exceeds 1:1, the culture solution required for the cells is not sufficiently converted, so that cell death may be induced. Therefore, it is preferred that the space part 130 and the sub well 120 have the above-described height range and height ratio.

Next, the sub wells 120 are formed at lower portion of each of the main wells 110 and include recessed parts 121 on a bottom surface thereof. As a particular aspect, the sub well 120 may include a plurality of recessed parts at lower portions of the main well 110.

The sub wells 120 included at the lower portion of the main well 110 have the same size and shape, thereby enabling spheroids and organoids to be produced under uniform conditions.

The sub well 120 may have an inclined surface formed so as to taper toward the recessed part 121. Specifically, the horizontal area of the upper portion of the sub well 120 may become smaller as it descends in the vertical direction. For example, the upper portion of the sub well 120 may be formed in an inverted pyramid shape. In the illustrated exemplary embodiment, the upper portion of the sub well 120 may be formed in a shape such as a pyramid shape or a funnel shape in which the horizontal area of the upper portion of the sub well 120 becomes smaller as it descends in the vertical direction.

In particular, the cell culture plate may produce a large amount of spheroids or organoids under uniform conditions by including a plurality of the sub wells 120 so as to have the same size and shape.

As a particular aspect, one main well 110 can include 4 to 25 sub wells 120 of the same size, and the entire microplate 100 may include 96 to 1,728 sub wells 120. Accordingly, the size can be controlled in the same precise manner.

Furthermore, the sub well 120 includes a recessed part 121, and a space is formed in the lower portion of the recessed part such that 3D spheroids or an organoids can be cultured in the recessed part 121. Specifically, the recessed part 121 may be in the form of the letter 'U', 'V', or ' 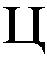 ', and for example, the recessed part 121 may be in the form of the letter 'U'.

The sub well 120 may have an upper end diameter ranging from 3.0 to 4.5 mm, or ranging from 3.5 to 4.3 mm, or 4 mm on average. Furthermore, the recessed part 121 may have an upper end diameter of 0.45 to 1.5 mm, or 0.5 to 1.0 mm or 0.5 mm on average.

Furthermore, a length ratio of the diameter of the sub well 120 to the diameter of the recessed part 121 may range from 1 :0.1 to 0.5, and preferably, a length ratio of the diameter of the sub well 120 to the diameter of the recessed part 121 may be 1:0.12.

When the upper end diameter of the recessed part 121 is less than 0.1 compared to the upper end diameter 1 of the sub well 120, a cell culture space of the recessed part 121 cannot be sufficiently provided, which may cause a problem in that cells escape even with a small force during the replacement of the culture solution, and when the upper end diameter of the recessed part 121 is exceeds 0.5 compared to the upper end diameter 1 of the sub well 120, a sufficient culture solution required for cells cannot be replaced, which may cause a problem in that it is difficult to stably culture cells.

Meanwhile, an inclination surface between the sub well 120 and the recessed part 121 may have an inclination angle (θ₂) of 40 to 50°, 42 to 48°, 43 to 47°, or an inclination angle (θ₂) of 45° on average, with respect to a wall of the main well.

The above-described sub well 120 has an advantage in that cells can be cultured at 100 to 1000 cells/well or less, and the spheroid size can be stably controlled.

Further, the main well 110 according to an exemplary embodiment of the present invention has an individual volume ranging from 100 to 300 µl, the recessed part 121 has an individual volume ranging from 20 to 50 µl, and an individual volume ratio of the main well 110 to the recessed part 121 is characterized by being 1:0.07 to 0.5 on average. Preferably, the main well according to an exemplary embodiment has an individual volume ranging from 250 to 300 µl, the recessed part has an individual volume ranging from 25 to 35 µl, and an individual volume ratio of the main well 110 to the recessed part 121 may be 1:0.11 on average.

Specifically, when the main well 110 has an individual volume less than 100 µl, a problem in that a sufficient culture solution cannot be accommodated during cell culture may occur, and when the individual volume exceeds 300 µl, culture efficiency may be reduced.

Furthermore, the recessed part 121 is a space in which cells are substantially cultured, and when the volume is less than 20 µl, the cell culture space is not sufficient, which may cause a problem in that cells escape, and when the volume exceeds 50 µl, a problem in that it is difficult to stably culture cells and the like may occur. Therefore, it is preferred that the main well 110 and the recessed part 121 have volumes in the above-described ranges.

Due to the above-mentioned configuration of the cell culture plate of the present invention, reprogramming into induced pluripotent stem cells occurs at high efficiency without including a hydrogel, that is, without coating the cell culture plate with a hydrogel, and the organoid is also formed well after the reprogramming.

The cell culture plate 10 according to an exemplary embodiment of the present invention includes a connector 200 for large-capacity and high-speed high content screening (HCS), which supports the well plate 100. Herein, the connector 200 for large-capacity and high-speed high content screening (HCS) refers to a connector 200 which is attached to a high content screening (HCS) system, and specifically, the connector may refer to a base 210 and a cover 220 in the present invention.

More specifically, the connector for large-capacity and high-speed high content screening (HCS) includes the base 210 equipped with fixing means 140 and 240 so as to be attached to and detached from a lower end of the well plate 100 and a cover 220 positioned on an upper portion of the well plate 100 to be coupled to the base 210. Moreover, the upper end of the base 210 and the lower end of the well plate 100 are characterized by including fixing means 140 and 240 that can be fixed so as to be attached to and detached from each other.

In this case, the base includes a convex part 240 for supporting the well plate 100, and the well plate 100 may include a concave part 140 facing the convex part 240 of the base 210. The well plate 100 may be fixed by the fixing means to uniformly capture images during screening.

The base may be formed of a polyethylene, polypropylene, polystyrene, polyethylene terephthalate, polyamide, polyester, polyvinyl chloride, polyurethane, polycarbonate, polyvinylidene chloride, polytetrafluoroethylene, polyether ether ketone or polyetherimide material, but is not limited thereto.

The well plates may be formed of a polydimethylsilicone, high-fat modified silicone, methylchlorophenylsilicone, alkyl-modified silicone, methylphenylsilicone, silicone polyester, or amino-modified silicone material, but is not limited thereto.

Meanwhile, when induced pluripotent stem cells are formed in the cell culture plate 10 of the present invention, Matrigel need not be used.

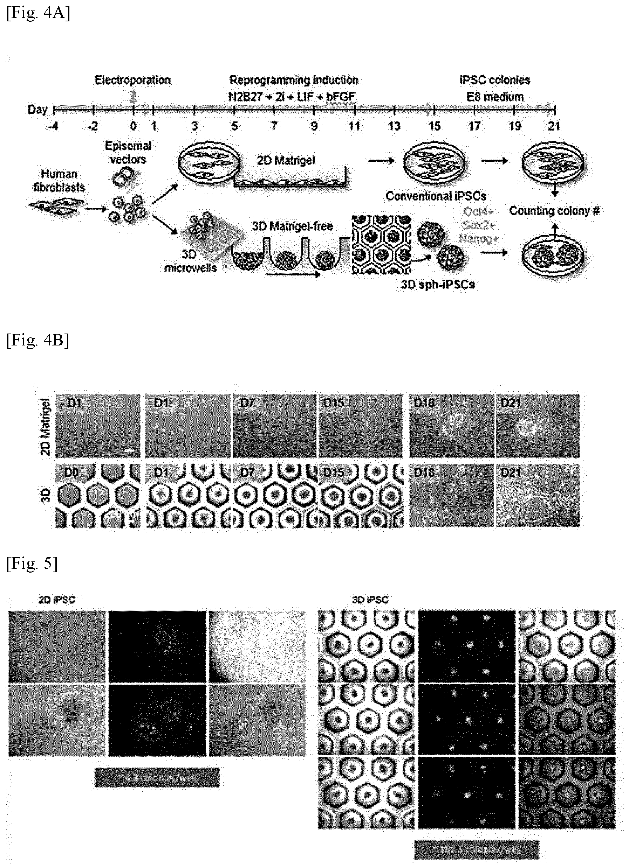

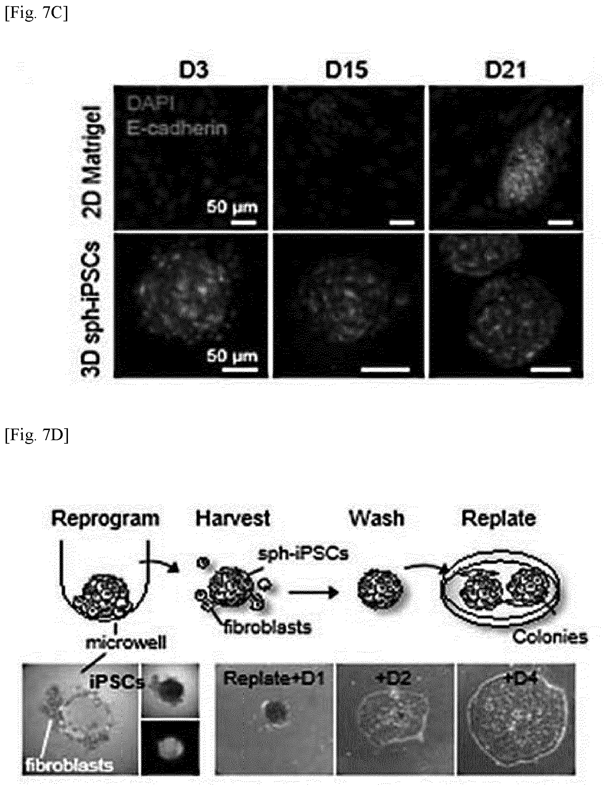

FIG 4 illustrates a comparison of a method of producing induced pluripotent stem cells using a 2D cell culture plate that uses Matrigel and a method of producing induced pluripotent stem cells using a 3D cell culture plate that does not require Matrigel according to the present invention. After somatic cells (fibroblasts) are cultured, induced pluripotent stem cells are produced by transfecting the fibroblasts with an episomal vector by electroporation to induce reprogramming. In the case of 2D Matrigel culture, the process of collecting a colony of induced pluripotent stem cells is complicated, and the yield is low. However, when the 3D culture plate of the present invention is used, there is no Matrigel, so that a number of single cells reprogrammed into induced pluripotent stem cells gather to form a spheroid, which is a 3D spherical cell aggregate. This spheroid can be easily separated from a 3D cell culture plate, and can be sub-cultured (FIG. 7D). That is, reprogramming efficiency is very high.

Further, as previously described, for the 3D cell culture plate used in the present invention, one main well 110 may include 4 to 25 sub wells 120 of the same size, and the entire microplate 100 may include 96 to 1,728 sub wells 120. Accordingly, it is possible to mass-produce induced pluripotent stem cells and spheroids thereof whose sizes can be controlled in the same precise manner.

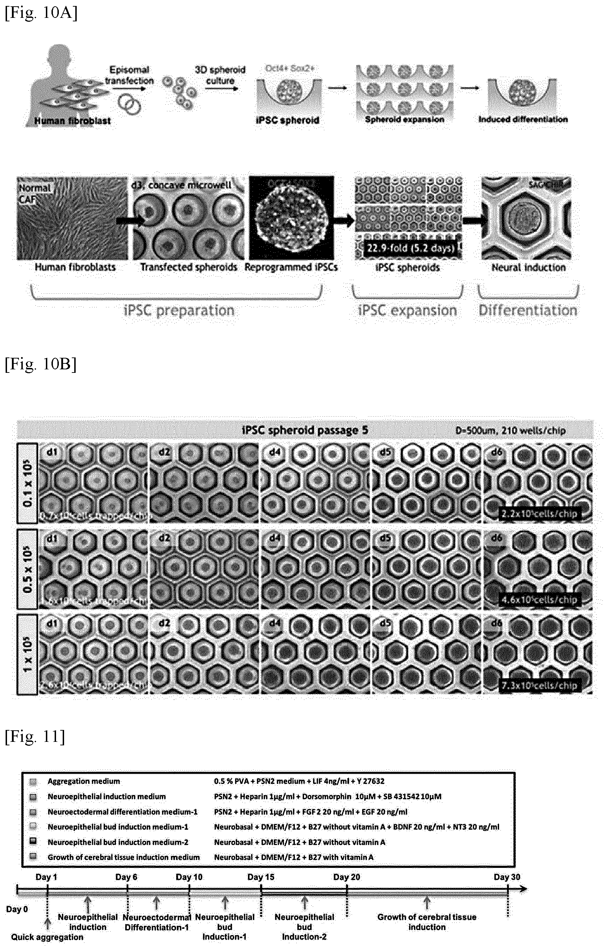

FIG 10 schematically illustrates a step of mass proliferating induced pluripotent stem cell spheroids obtained in the step of reprogramming induced pluripotent stem cells and inducing cell differentiation. When the 3D cell culture plate of the present invention is used, it can be seen that the proliferation rate of induced pluripotent stem cells is very high. In addition, when spheroids are separated into single cells and the single cells are plated again, and then subcultured, hundreds to thousands of uniformly sized monoclonal spheroids are produced, so that an induced pluripotent stem cell spheroid bank may also be produced. Cell differentiation may be induced using this bank, and the present invention produces brain organoids by inducing neural differentiation.

### [Advantageous Effects]

According to the production method of the present invention, it is possible to produce induced pluripotent stem cells with enhanced reprogramming efficiency without the need for a hydrogel, and then produce brain organoids using the induced pluripotent stem cells. In addition, the brain organoid according to the present invention is an ultra-small brain organoid having a size of 13 mm or less, which is extremely small.

### [Description of Drawings]

FIG 1A is a front view of a cell culture plate according to an exemplary embodiment of the present invention, and FIG 1B is a cross-sectional view of the cell culture plate according to an exemplary embodiment of the present invention.
FIG 2 is a view illustrating, in detail, a main well formed in the cell culture plate according to an exemplary embodiment of the present invention.
FIG 3 is a view illustrating a well plate, a base, and a cover of the cell culture plate according to an exemplary embodiment of the present invention ((A) a cover, (B) a base, and (C) a fixing means of a microplate and a base).
FIG 4A schematically illustrates processes of producing induced pluripotent stem cells according to an exemplary embodiment of the present invention and a comparative example, and FIG 4B is a set of images illustrating the generation of induced pluripotent stem cells according to an exemplary embodiment of the present invention and a comparative example (In all of the drawings below FIG 4, for convenience of description, the 3D cell culture plate of the present invention is not accurately displayed, but is displayed in a U shape for convenience.)
FIG 5 is a set of images of an exemplary embodiment (3D iPSC) of the present invention and a comparative example (2D iPSC).
FIG 6 is an alkaline phosphatase (AP) stained image of an exemplary embodiment (3D sph-iPSC) of the present invention and a comparative example (2D Matrigel).
FIG 7A is an AP image (D4, D9, D15, D21) over time, FIG 7B compares the number of colonies, FIG 7C is an E-cadherin expression result, and FIG 7D illustrates the process of forming a spheroid of iPSCs.
FIG 8A is a result of showing the size distribution of spheroids (colonies) as a result of 3D culture in the related art and culture according to an exemplary embodiment of the present invention, and FIG 8B is the expression result of a reprogramming factor (pluripotency marker).
FIG 9 illustrates the expression results of pluripotency markers of iPSCs according to an exemplary embodiment of the present invention.
FIG 10 schematically illustrates a step of mass proliferating induced pluripotent stem cell spheroids obtained in the step of reprogramming induced pluripotent stem cells and inducing cell differentiation.
FIG 11 schematically illustrates the process of forming the brain organoid according to an exemplary embodiment of the present invention.
FIG 12A is a set of brain organoid images over time, which are produced according to an exemplary embodiment of the present invention, FIG 12B illustrates a set of images of brain organoids cultured at a uniform size while being mass-cultured in one cell culture plate according to an exemplary embodiment of the present invention, and FIG 12C is a graph illustrating changes in size of a brain organoid over time.
FIG 13 is a set of images of brain organoids formed in the absence of Matrigel according to an exemplary embodiment of the present invention.
FIG 14A is a stained image of brain organoids produced by the method of Non-Patent Document 3, and FIG 14B is a stained image of brain organoids formed by an exemplary embodiment of the present invention.
FIG 15A illustrates the results of brain organoid immunostaining analysis, and FIG 15B illustrates the results of brain organoid gene expression analysis.

### [Modes of the Invention]

Since the present invention may be modified into various forms and include various exemplary embodiments, specific exemplary embodiments will be illustrated in the drawings and described in detail in the Detailed Description. However, the description is not intended to limit the present invention to the specific exemplary embodiments, and it is to be understood that all the changes, equivalents, and substitutions belonging to the spirit and technical scope of the present invention are included in the present invention. When it is determined that the detailed description of the related publicly known art in describing the present invention may obscure the gist of the present invention, the detailed description thereof will be omitted.

### [Examples]

### Example 1. Experimental methods

### 1-1: Culture of fibroblasts and production of induced pluripotent stem cells

The German federal authorities/RKI: AZ 1710-79-1-4-41 E01 (F134), which is a human fibroblast line, was cultured in a DMEM containing 10% FBS (fetal bovine serum, Invitrogen, USA) and 1 mM L-glutamine (Invitrogen, USA) in a 35 mm or 100 mm Petri dish. The cultured fibroblasts were reprogrammed by being transfected (NeonTM transfection system) with an episomal iPSC reprogramming vector (EP5TM kit: Cat. No. A16960. Invitrogen, Carlsbad, CA, USA) by electroporation. The electroporation was performed under the conditions of 1,650 V, 10 ms, and 3 pulses.

As illustrated in FIG 4A, the transfected fibroblasts were inoculated in a 3D cell culture plate of the present invention (without Matrigel, the Example), a 2D 12-well plate (coated with Matrigel, Comparative Example 1) and a commercialized product Addgene (Comparative Example 2, coated with Matrigel, not illustrated in FIG 4A), and cultured in an N2B27 medium (containing bFGF). After the fibroblasts were cultured for 15 days, the medium was replaced with an Essential 8TM medium. After 15 days, the number of colonies in the Example and the Comparative Examples were confirmed by plating 3D iPSCs of the Example (3D cell culture plate) on a 12-well plate which is a 2D plate.

### 1-2: Reprogramming efficiency analysis of fibroblasts

According to the alkaline phosphatase staining kit manual (System Biosciences, USA), reprogrammed cells were washed twice with PBS, fixed with 4% paraformaldehyde, then stained with a Blue-color AP solution, washed twice with PBS, and then it was observed under an optical microscope whether the colonies were stained. The number of stained colonies was counted and quantified.

Images of the cultured cells in the Example and the Comparative Examples were captured, and the sizes of cell spheres were compared. Spheroids were subjected to imaging by an automated plate device, and in this case, the device was allowed to perform imaging by automatically focusing. Image size analysis was performed using a macro program of a program called Imaged (related to FIGS. 5, 6 and 7).

### 1-3: Optimization of 3D culture method of induced pluripotent stem cells

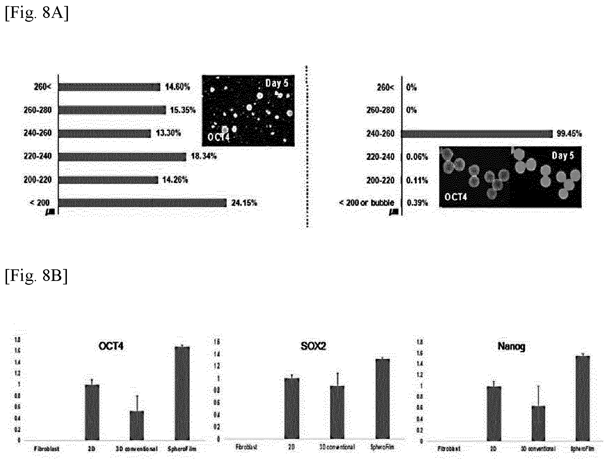

Images of the 3D induced pluripotent stem cells cultured in the Example and the Comparative Examples were captured, and accordingly, the sizes of the cell spheres were compared and measured (FIG. 8A). An external inspection company (Cell Bio CEFO, Korea) was commissioned to test the results of the images, and this test was performed as a blind test.

### 1-4: Immunostaining

Reprogrammed cells were fixed with 4% paraformaldehyde at room temperature for 20 minutes. After the fixed cells were reacted with PBS containing 1% BSA and 0.5% Triton X-100 at room temperature for 1 hour, the cells were treated with each of primary antibodies Oct4 (1:100, SantaCruz, CA, USA), Sox2 (1:100, Cell Signaling, Danvers, MA, USA), Nanog (1:200, Cosmo Bio, Koto-Ku, Japan), and E-cadherin (1:200, Abcam), and reacted with FITC-conjugated goat anti-rabbit IgG or anti-mouse IgG (1:100, Invitrogen, Carlsbad, CA) as a secondary antibody. Fluorescent images were analyzed under a fluorescence microscope (Olympus, Shinjuku, Tokyo, Japan). DAPI was used as a nuclear staining solution.

### 1-5: qPCR

Total RNA was extracted from fibroblasts and reprogrammed cells using an RNA minikit (Qiagen, Inc.), and then converted to cDNA using the Accupower RT mix reagent (Bioneer Corp., Seoul, Korea). qPCR was performed using Real-time PCR FastStart Essential DNA Green Master Mix (Roche, Indianapolis, IN, USA). The primer sequences used in the present invention are as follows in Table 1.

**[Table 1]**

| Genes | Primer sequences (5'-3') | |
|---|---|---|
| hCOL1Al | forward | ATGACTATGAGTATGGGGAAGCA |
| | reverse | TGGGTCCCTCTGTTACACTTT |
| hOCT4 | forward | AATTTGTTCCTGCAGTGCCC |
| | reverse | AGACCCAGCAGCCTCAAAAT |
| hNANOG | forward | GGATCCAGCTTGTCCCCAAA |
| | reverse | TGCGACACTCTTCTCTGCAG |
| hSOX2 | forward | CGGAAAACCAAGACGCTCAT |
| | reverse | GTTCATGTGCGCGTAACTGT |
| hLIN28 | forward | TTCGGCTTCCTGTCCATGAC |
| | reverse | CCGCCTCTCACTCCCAATAC |

### 1-6: Production and analysis of brain organoids

3D brain organoids were produced using reprogrammed iPSC cells. Brain organoids were cultured by initially seeding 9000 iPSC cells per well on a cell culture plate according to the present invention having a diameter of 3 mm and adjusting the composition of the culture solution. The composition of the culture solution in each culturing step followed the paper of M.A. Lancaster (Non-Patent Document 1), but the brain organoids were cultured without using Matrigel (see FIG 11).

The characteristics of the produced brain organoids were analyzed by the immunostaining method and gene expression analysis. Organoids of 1 mm or more were fixed using 4% PFA for immunostaining, and then sufficiently immersed in 15% and 30% sucrose. And then, a block was manufactured by transferring the brain organoids to an O.C.T compound and then quick-freezing the O.C.T compound. Organoids were cut to a thickness of about 10 to 15 um using a cryotome, and then stained using an existing 2D immunostaining method. (FOXG1 (1:500, Abcam), MAP2 (1:500, Abcam)).

RNA was extracted from cultured brain organoids using an RNA extraction kit (RNEasy plus kit, Qiagen), and cDNA was synthesized (High-capacity RNA-to-cDNA kit, Stepone plus). A gene expression level of the corresponding gene was analyzed by RT-PCR using the primers designed as shown in Table 2.

**[Table 2]**

| Genes | | Primer sequences (5'-3') |
|---|---|---|
| hOCT4 | Forward | GCCACACGTAGGTTCTTGAA |
| | Reverse | ATCGGCCTGTGTATATCCCA |
| hTBR1 | Forward | CCAATCTCTTCTCCCAGGGA |
| | Reverse | CTAGAACCTGAACACTCGCC |
| hCtip2 | Forward | CCACTTGGCATTAGAGGGTC |
| | Reverse | TTGCAGGGCTGAGTTACAAG |

### Example 2. Confirmation of stem cell reprogramming efficiency

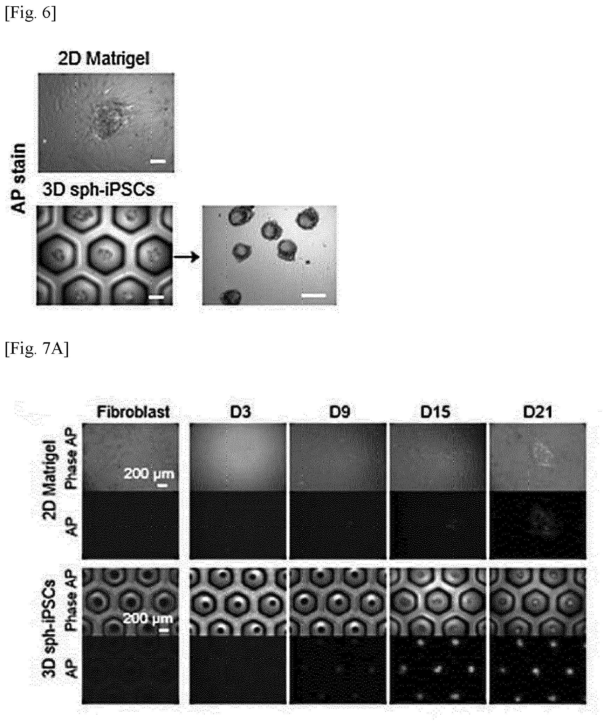

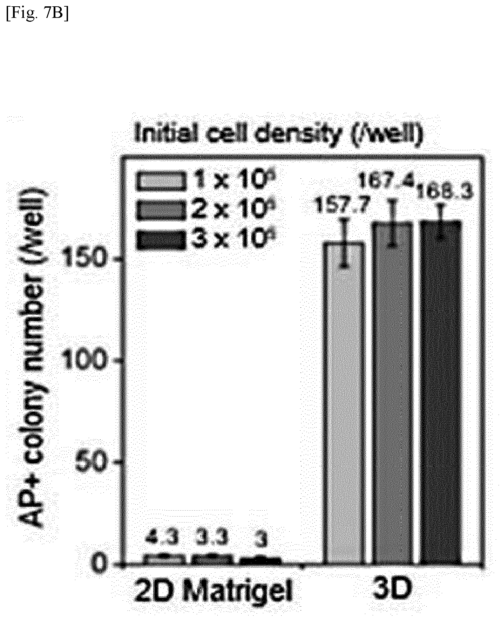

Referring to FIG 4B, it can be seen that in the case of 2D culture, a small amount of colonies begin to be formed only at D15. After iPSC reprogramming was induced up to D15, 3D iPSCs were plated on a 2D plate, and the number of colonies in Comparative Example 1 and Example 1 was compared, and as a result, the difference in the number of colonies formed was large. It can be seen that the iPSC reprogramming yield of the Example is high because the cells that are well differentiated into iPSCs form a colony. Referring to FIGS. 5 and 7B, it can be seen that the difference in the number of colonies is very large between the 2D culture (Comparative Example 1) and the 3D culture (the Example). Referring to FIG 6, it can be seen that as a result of alkaline phosphatase (AP) staining, the reprogramming efficiency is very high in 3D iPSC spheroids (the Example, 3D sph-iPCSs). Further, referring to FIGS. 6 and 7A, when 2D Matrigel (Comparative Example 1) and 3D iPSC spheroids (the Example, 3D sph-iPCSs) are compared with each other, the images appear uniform and clear, showing that the 3D cell culture plate of the present invention is capable of large-scale image analysis.

Referring to FIG 7C, it can be seen that the reprogramming efficiency in the 3D cell culture plate is very good. In addition, referring to FIG. 7D, it can be seen that since the present invention does not use Matrigel, a large number of single cells reprogrammed into iPCSs gather to form a spheroid, which is a spherical cell aggregate, and these spheroids can be easily separated from the 3D cell culture plate and re-plated. That is, reprogramming efficiency is very high.

FIG 8 compares the 3D culture in the related art of Comparative Example 2 with the 3D culture of the Example of the present invention (SpheroidFilm in FIG 8B). The 3D culture in the related art is not uniform in size and has a relatively low expression level of oct4. However, the present invention is very uniform in size (99.45%) and has a very high reprogramming factor expression level. That is, the present invention is effective in stem cell culture even when compared to the 3D culture in the related art, and can increase the efficiency of reprogramming somatic cells into induced pluripotent stem cells. Furthermore, a uniform size means that standardized induced pluripotent stem cells and stem cells can be three-dimensionally mass-produced in the form of a spheroid.

### Example 3. Analysis of characteristics of stem cells

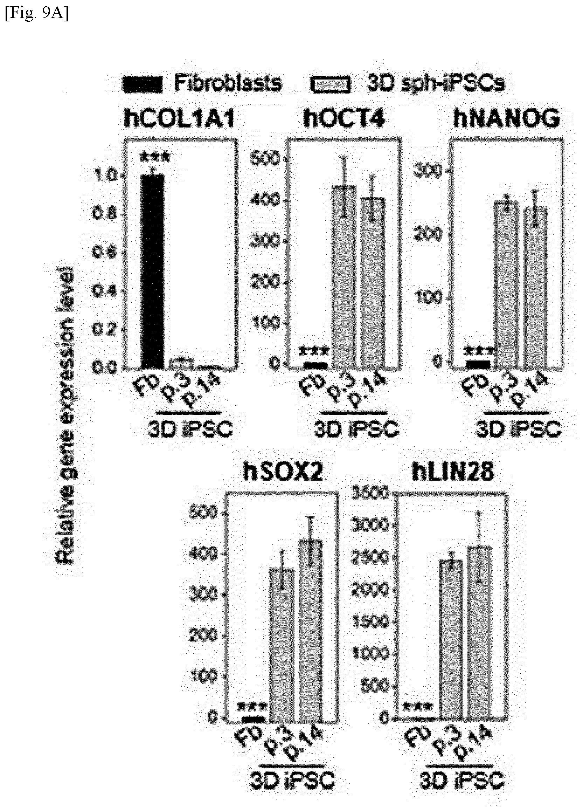

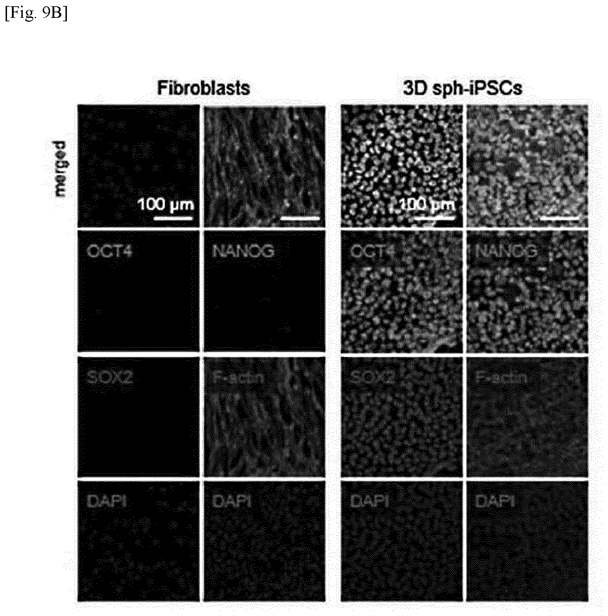

Referring to FIG 9, it can be seen that the iPSCs produced according to the present invention have very high expression of pluripotency markers.

### Example 4. Production and characteristic analysis of brain organoids

### 4-1: Production of brain organoids

Referring to FIG 10, it can be seen that when the iPSCs produced by the present invention are subcultured, the iPSCs can be mass-proliferated. FIG 10 also shows that the iPSCs thus produced can be used for the production of brain organoids.

Referring to FIG 11, the method of producing brain organoids in each step is described. The method is divided into a step of making stem cells into an embryonic body, a step of inducing neuroectodermal cells, a step of differentiating the neuroectodermal cells into a neuroectodermal tissue, a step of enhancing a neuroectodermal bud, and a final step of differentiating into brain organoids from the neuroectodermal bud. After the process of the brain organoids produced in this manner, the brain organoids are subjected to a process of being cultured in a differentiation medium.

### 4-2: Characteristic analysis of brain organoids

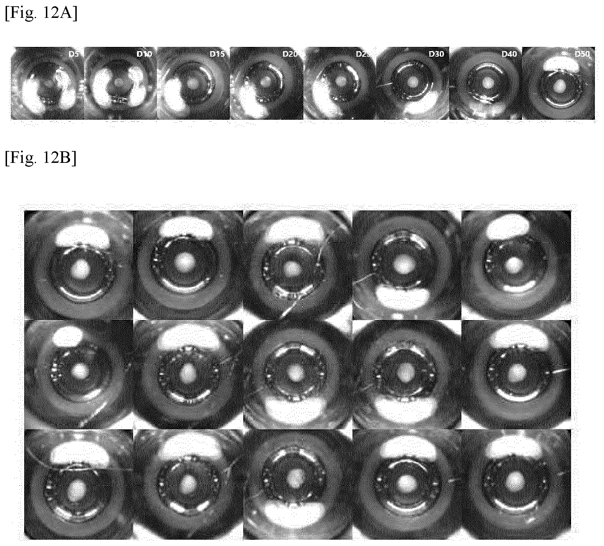

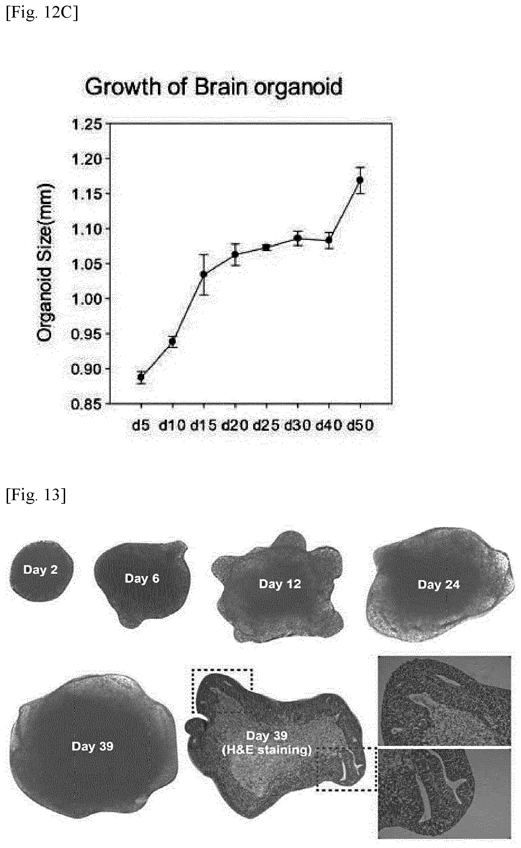

Referring to FIG 12, it can be seen that the brain organoid produced by the present invention is produced while having a uniform size. FIG 12B is a photograph supporting the high-speed, large-capacity imaging of the cell culture plate of the present invention. The organoid production method according to the present invention is economical because Matrigel is not used, and has an advantage of not taking up a large space because the organoid is a mini-brain organoid.

FIG 13 is a photograph of a brain organoid culture, and it can be confirmed that various organs are randomly mixed and thus do not appear. Within 2 days after the seeding of initial cells, it was confirmed that a uniformly sized embryonic body having a diameter of about 500 mm was formed, and that a transparent neuroepithelium was formed on the outside of the organoid as nerve differentiation progressed (Day 12 of culture). As a result of confirming the cross section of the brain organoid cultured for about 40 days by H&E staining, neural rosette structures could be found on the outside thereof.

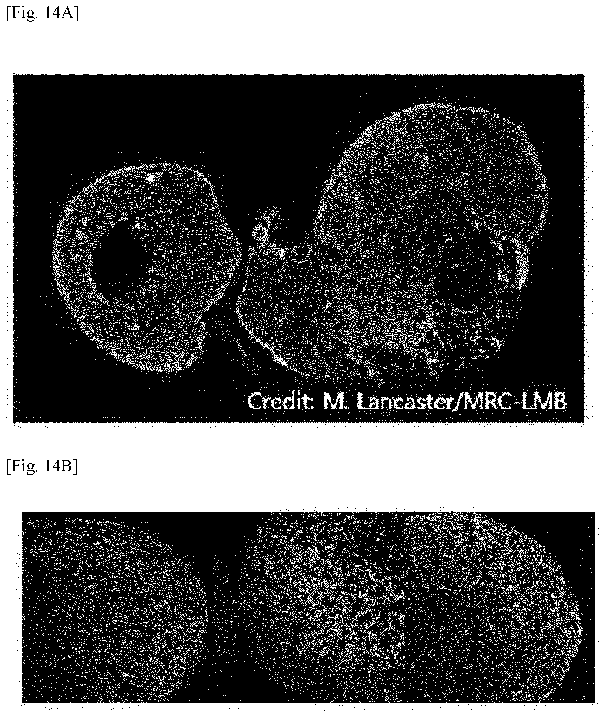

Referring to FIG 14A, it can be seen that a cortical plate and a neuron position are different, and that the neuron grows randomly even within one organoid. However, referring to FIG 14B, it can be confirmed that the cortical plate and the neuron position are uniform, and that there is a uniform ventricular zone in one organoid and multiple organoids, respectively.

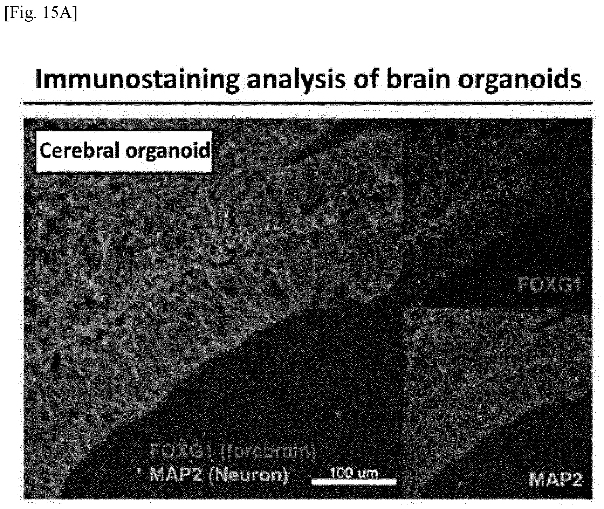

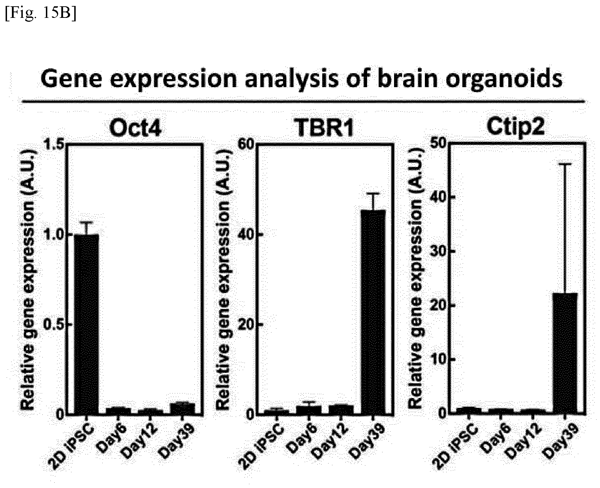

FIG 15 illustrates the results of analyzing brain organoids for 40 days in the absence of Matrigel according to an exemplary embodiment of the present invention. As a result of immunostaining analysis (A), it was confirmed that Foxg1 was expressed in Forebrain. As a result of gene expression analysis (B), it was confirmed that the expression of Oct4, which is a pluripotency marker of iPSCs, decreases with differentiation, and that the expression of TBR1 and Ctip2, which are neural markers expressed in a deep layer, increases.

Although a specific part of the present invention has been described in detail, it will be obvious to those skilled in the art that such a specific description is just a preferred embodiment and the scope of the present invention is not limited thereby. Accordingly, the substantial scope of the present invention will be defined by the appended claims and equivalents thereof.

### [Description of Reference Numerals and Symbols]

100: Well plate
101: Step
110: Main well
120: Sub well
121: Recessed part
130: Space part
140: Concave part
200: Connector for large-capacity and high-speed HCS
210: Base
220: Cover
240: Convex part

## Claims

1. A method of producing brain organoids, the method comprising:
i) culturing somatic cells;
ii) preparing a hydrogel-free 3D cell culture plate for producing induced pluripotent stem cells;
iii) producing induced pluripotent stem cells by reprogramming the cultured somatic cells into the induced pluripotent stem cells in the hydrogel-free 3D cell culture plate;
iv) isolating the induced pluripotent stem cells from the 3D cell culture plate of step iii);
v) preparing a 3D cell culture plate which is not coated with a hydrogel for forming brain organoids; and
vi) forming brain organoids by culturing the isolated induced pluripotent stem cells in a hydrogel-free 3D cell culture plate,
wherein the 3D cell culture plate comprises:
a well plate comprising a plurality of main wells and a plurality of sub wells formed at lower portions of the main wells to be injected with a cell culture solution and comprising recessed parts on a bottom surface thereof; and a connector for large-capacity and high-speed high content screening (HCS), which supports the well plate, and
the connector for high content screening (HCS) comprises a base equipped with a fixing means so as to be attached to and detached from a lower end of the well plate and a cover positioned on an upper portion of the well plate to be coupled to the base,
the main well has a step formed so as to be tapered at a predetermined site, and the step has an inclination angle (θ) ranging from 10 to 60° with respect to a wall of the main well.

2. The method of claim 1, wherein the forming of the brain organoids comprises,
after making the induced pluripotent stem cells into an embryonic body,
inducing neuroepithelial cells by adding a neuroepithelial induction medium to the aggregated induced pluripotent stem cells;
differentiating the neuroepithelial cells into a neuroectodermal tissue by adding a neuroectodermal differentiation medium thereto;
proliferating a neuroepithelial bud by adding a neuroepithelial bud induction medium to the neuroectodermal tissue; and
forming a brain tissue by adding a brain tissue induction medium to the proliferated neuroepithelial bud.

3. The method of claim 1, wherein the hydrogel is an extracellular matrix-based hydrogel.

4. The method of claim 3, wherein the extracellular matrix-based hydrogel is Matrigel.

5. The method of claim 1, wherein the brain organoid has a size of 0.8 to 1.3 mm.

6. The method of claim 1, wherein the brain organoid has a size of 1 mm or less.

7. The method of claim 1, wherein the sub well has an inclined surface formed so as to taper toward the recessed part,
the sub wells have an upper end diameter ranging from 3.0 to 4.5 mm,
the recessed parts have an upper end diameter ranging from 0.45 to 1.5 mm,
an inclined surface (θ₂) between the sub well and the recessed part ranges from 40 to 50°, and
a length ratio of the diameter of the sub wells to the diameter of the recessed parts ranges from 1:0.1 to 0.5.

8. The method of claim 1, wherein the main well has an individual volume ranging from 100 to 300 µl,
the recessed part has an individual volume ranging from 20 to 50 µl, and
an individual volume ratio of the main well to the recessed part is 1:0.1 to 0.5 on average.

9. The method of claim 1, wherein the main well comprises a space part between the step and the sub well,
the space part has a height (aₕ) ranging from 2.0 to 3.0 mm on average,
the sub well has a height (bₕ) ranging from 1.0 to 2.0 mm on average, and
a height ratio (aₕ:bₕ) of the space part to the sub well ranges from 1:0.3 to 1.
